(19) Europäisches Patentamt
European Patent Office
Office européen des brevets

(11) Publication number: 0 269 339
A2

(12) # EUROPEAN PATENT APPLICATION

(21) Application number: 87310102.6

(22) Date of filing: 16.11.87

(51) Int. Cl.[4]: C07H 15/252

(30) Priority: 17.11.86 CA 523113

(43) Date of publication of application:
01.06.88 Bulletin 88/22

(84) Designated Contracting States:
AT BE CH DE ES FR GB GR IT LI LU NL SE

(71) Applicant: ABI BIOTECHNOLOGY INC.
A-1150 Waverley Street
Winnipeg Manitoba R3T OP4(CA)

(72) Inventor: Wong, Chiu Ming
31 Folkestone Boulevard
Winnipeg Manitoba R3P 0B4(CA)

(74) Representative: Goldin, Douglas Michael et al
J.A. KEMP & CO. 14, South Square Gray's Inn
London WC1R 5EU(GB)

(54) **Novel daunomycin and adriamycin sulfono and sulfoxo.**

(57) This invention provides novel compounds of the formula (I):

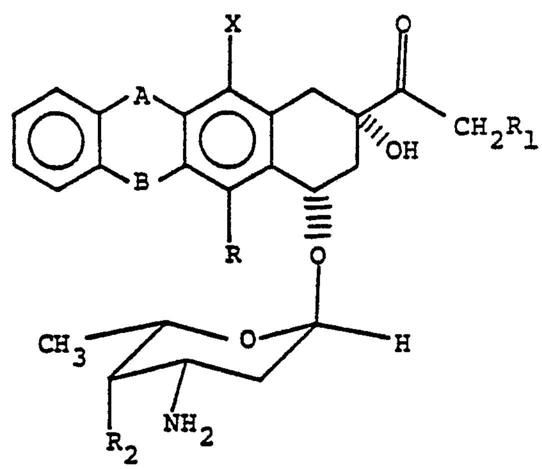

(I)

including all stereoisomers thereof, and pharmaceutically acceptable salts thereof, wherein, of A and B, one is C=O and the other is S⩳Y; R is hydrogen, hydroxy or methoxy; $R_1$ is hydrogen or hydroxy; $R_2$ is hydrogen or hydroxy; X is hydrogen, hydroxy or methoxy and Y is O or $O_2$; provided that R and X do not both stand for methoxy.

These compounds show potent growth inhibition activity against P-388 leukemia cells and are thereofore potentially useful in the treatment of cancer.

EP 0 269 339 A2

Xerox Copy Centre

## NOVEL DAUNOMYCIN AND ADRIAMYCIN SULFONO AND SULFOXO ANALOGUES

This invention relates to novel daunomycin and adriamycin sulfono and sulfoxo analogues and more particularly, it relates to 4-demethoxy analogues which show potent growth inhibition activity against P-388 leukemia cells and are therefore potentially useful in the treatment of cancer.

According to the invention we provide novel compounds of the formula (I):

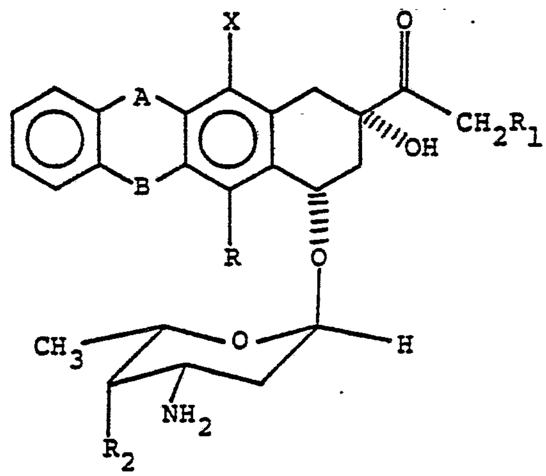

(I)

including all stereoisomers thereof, and pharmaceutically acceptable salts thereof, wherein, of A and B, one is >C=O and the other is >S≈Y; R is hydrogen, hydroxy or methoxy; $R_1$ is hydrogen or hydroxy ; $R_2$ is hydrogen or hydroxy; X is hydrogen, hydroxy or methoxy and Y is O or $O_2$; provided that R and X do not both stand for methoxy.

Preferred compounds are those of the formula (II):

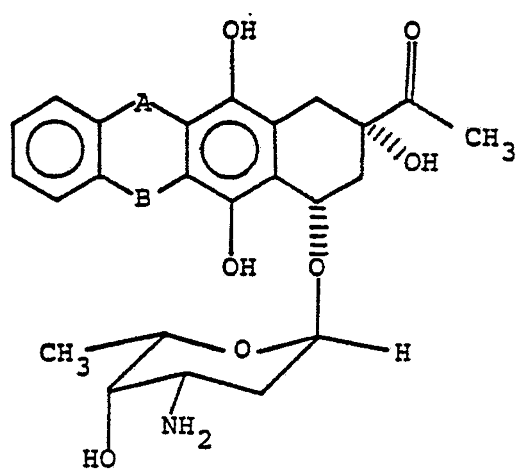

(II)

wherein A and B have the meaning stated above.

Particularly useful compounds are 4-demethoxy-5-sulfono-and 4-demethoxy-12-sulfono-daunomycin of the formulae (III) and (IV), respectively:

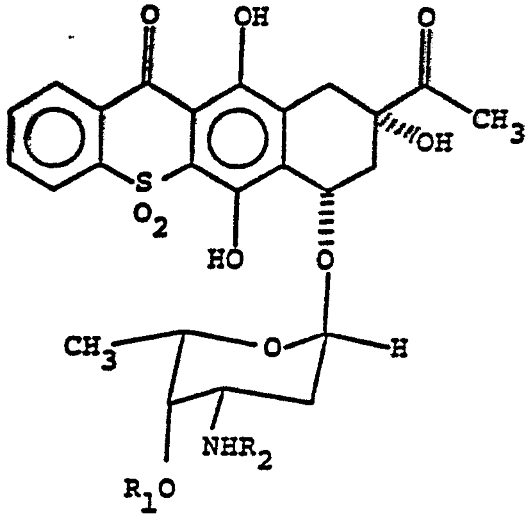

(III) $R_1=R_2=H$
(IIIa) $R_1$=p-nitrobenzoyl
$R_2$=trifluoroacetyl

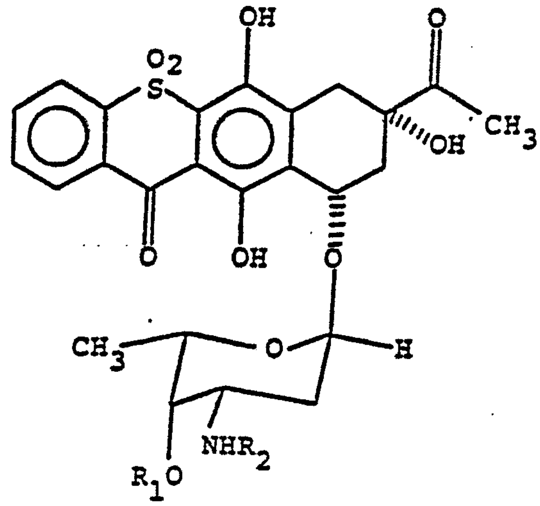

(IV) $R_1=R_2=H$
(IVa) $R_1$=p-nitrobenzoyl
$R_2$=trifluoroacetyl

and the corresponding 4-demethoxy-5-sulfoxo and 4-demethoxy-12-sulfoxo analogues of the formulae (V), (VI), (VII) and (VIII), respectively:

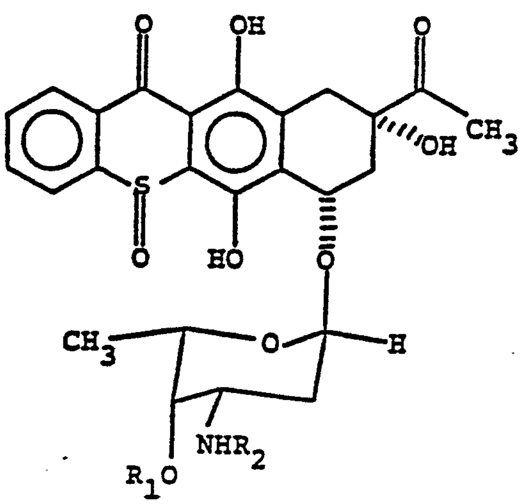

(V) $R_1=R_2=H$
(Va) $R_1$=p-nitrobenzoyl
$R_2$=trifluoroacetyl

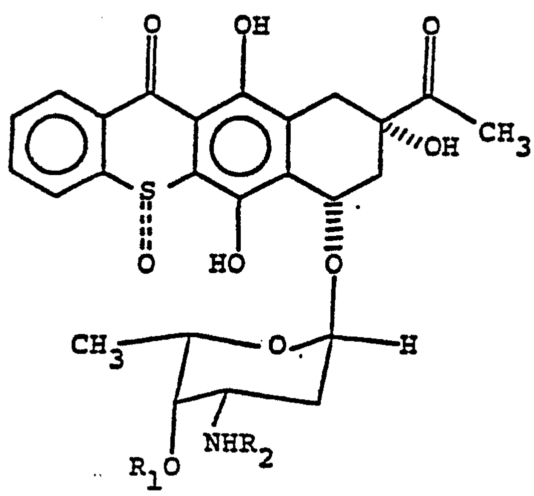

(VI) $R_1=R_2=H$
(VIa) $R_1$=p-nitrobenzoyl
$R_2$=trifluoroacetyl

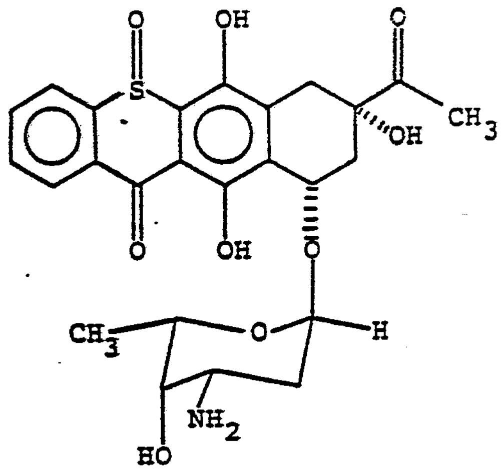

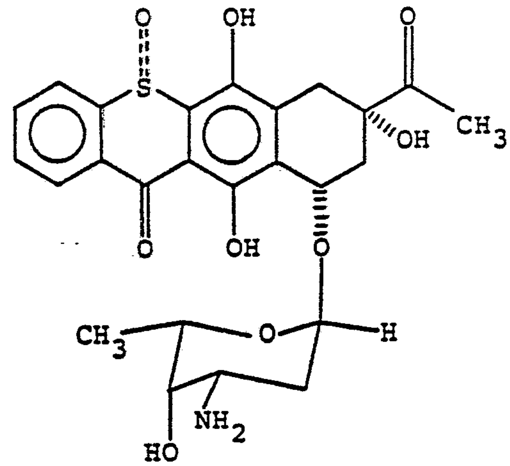

(VII) (VIII)

The 11-deoxy compounds corresponding to the above compounds are also valuable and have the formulae given below:

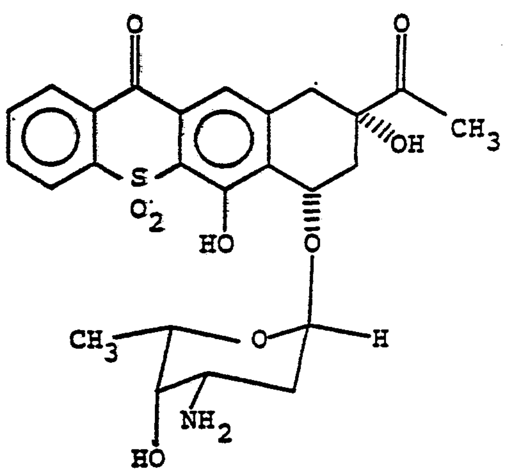

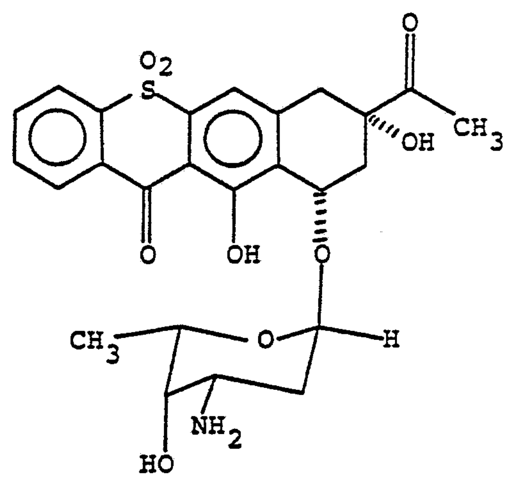

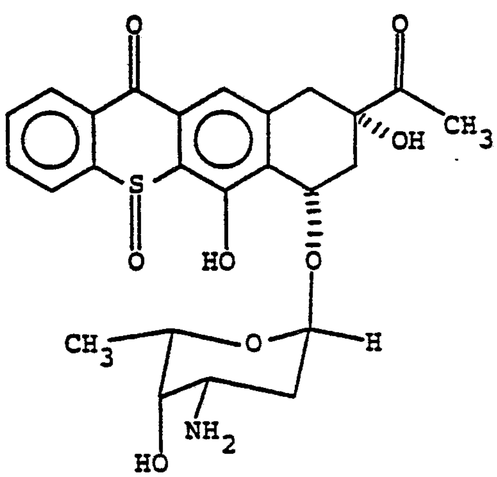

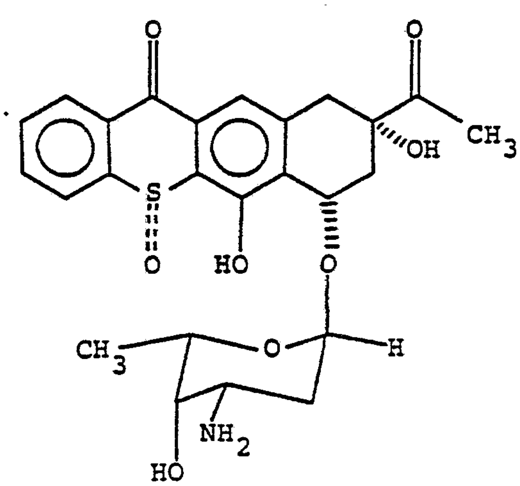

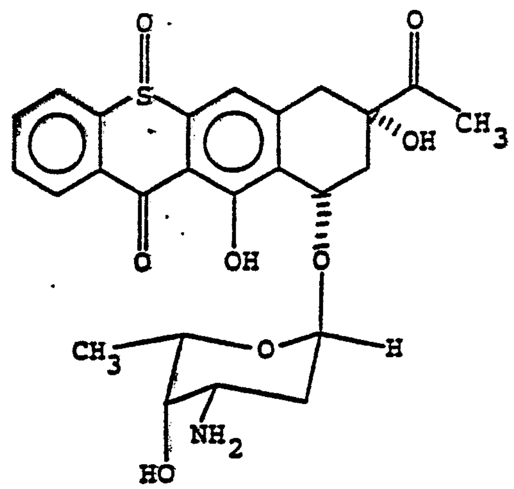

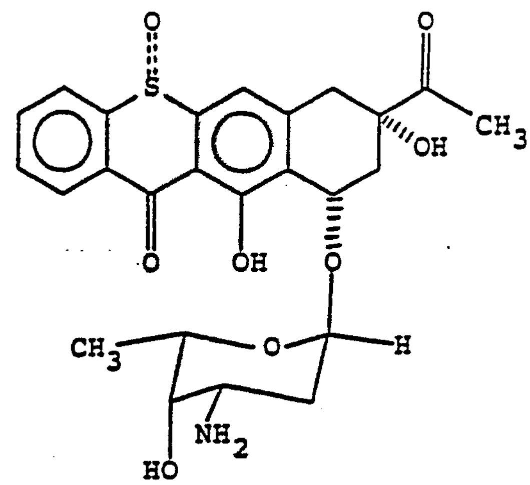

Interesting mono-methyl derivatives are 6-methoxy and 11-methoxy compounds of the formulae (IX) and (X), respectively:

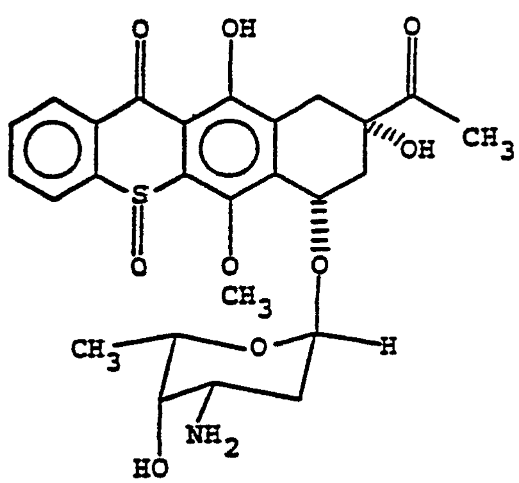

(IX)

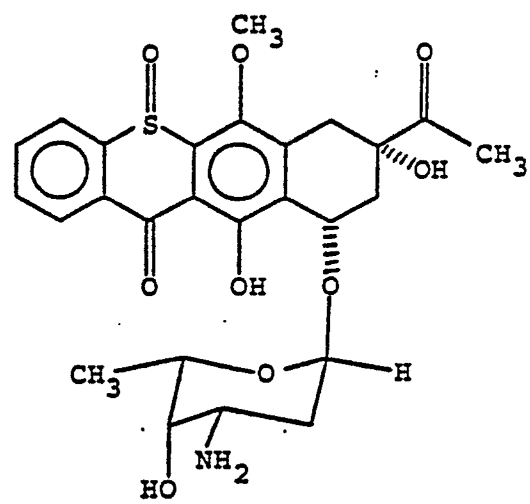

(X)

According to a further feature of the invention we produced a process for the preparation of these novel compounds which comprises reacting an aglycone of the formula (XI) or (XII):

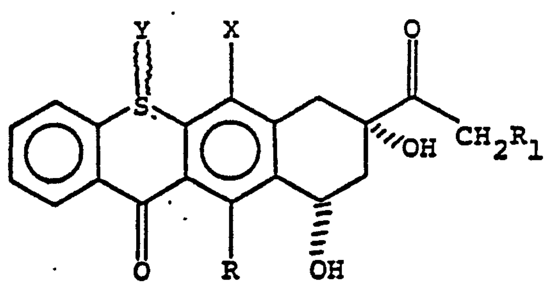

(XI)

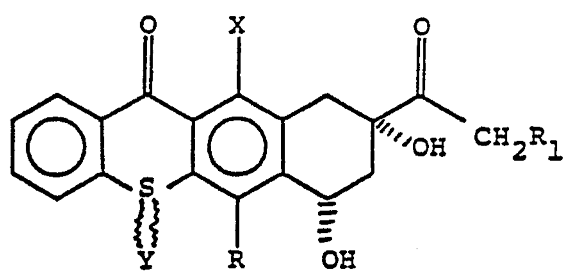

(XII)

with a daunosamine derivative of the formula:

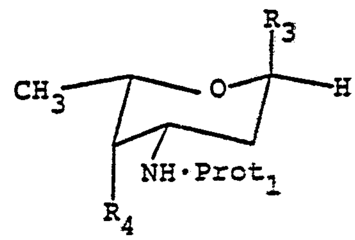

wherein R, $R_1$, X and Y have the meaning stated above, $R_3$ is a leaving group and $R_4$ is hydrogen or O•$Prot_2$, wherein $Prot_1$ and $Prot_2$ are protecting groups and subsequently removing any protecting group or groups to provide the final product.

As a suitable leaving group there may be mentioned a halogen, for example chlorine. A suitable protecting group $Prot_1$ may be a trifluoroacetyl group and a suitable protecting group $Prot_2$ may be a p-nitrobenzoyl group. A useful daunosamine derivative is 1-chloro-3-N-trifluoroacetyl-4-O-p-nitrobenzoyl-daunosamine of the formula:

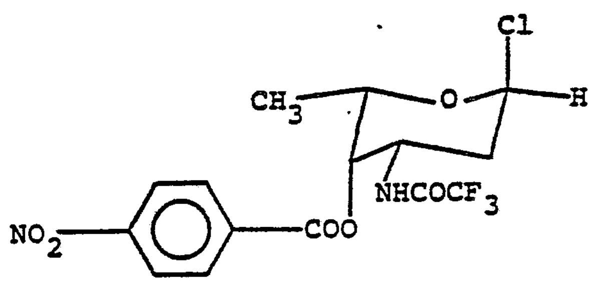

The reaction of the aglycone with the daunosamine derivative may be conveniently carried out in the presence of a diluent or solvent, for example methylene dichloride, in the presence of silver trifluoromethanesulfonate. The removal of any protecting group or groups may be carried out in an alkaline medium for example in the presence of 1% sodium hydroxide in tetrahydrofuran solution.

The aglycones used as intermediates may generally be prepared by various processes according to the particular isomer required and whether or not a sulfono compound or a sulfoxide compound is to be prepared.

Reaction of 2-acetyl-5,8-dimethoxytetralin (XIII) with either a) 2-chlorosulfenylbenzoyl chloride in the presence of stannic chloride in 1,2-dichloroethane or b) 2,2'-dithiobenzoic acid in concentrated sulfuric acid provides a mixture of (XIV) and (XV):

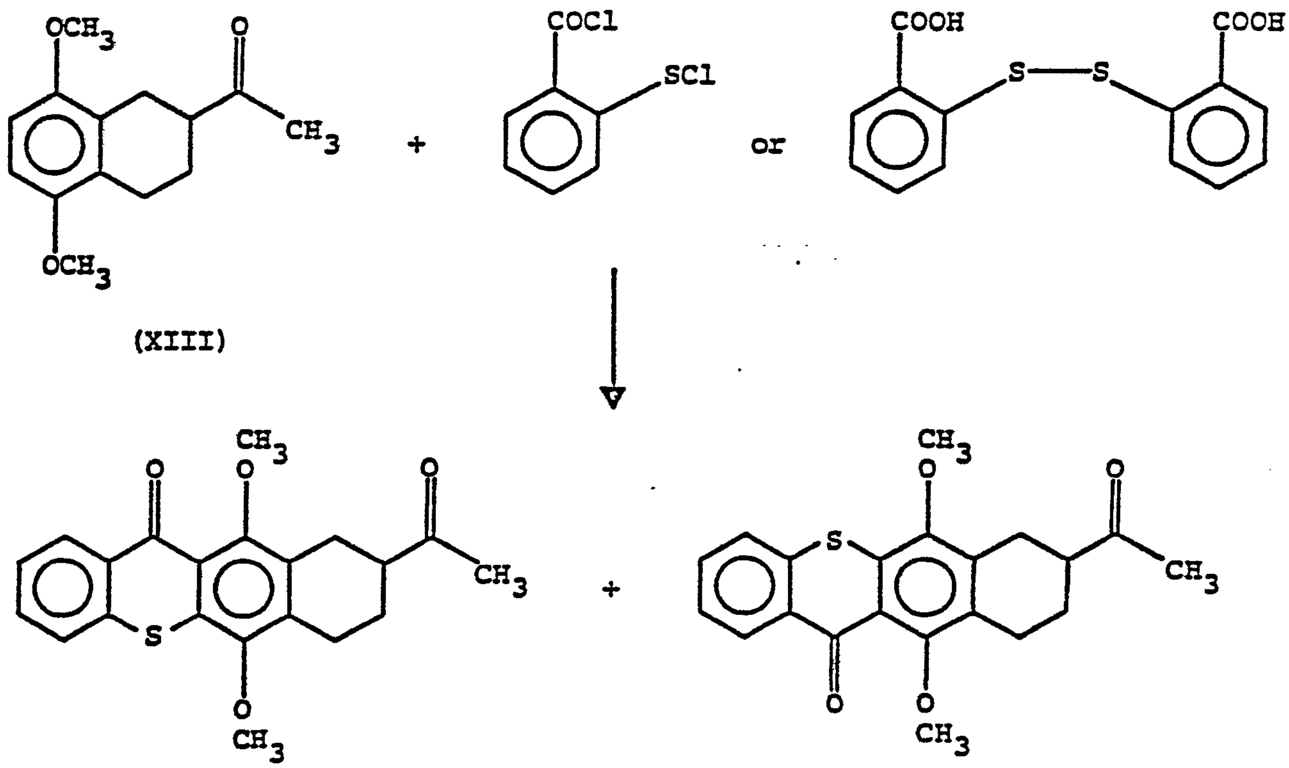

(XIII) + or

(XIV) + (XV)

Compound (XIV) can be oxidized with gaseous oxygen in the presence of potassium t-butoxide and trimethyl phosphite in a mixture of dimethylformamide and t-butanol to the corresponding dihydroxy compound (XVI). The latter can then be further oxidized, for example either with peroxybenzoic acid or with a mixture of selenium dioxide and hydrogen peroxide. When using 2.5 moles of peroxybenzoic acid, there is obtained the sulfono compound (XVII) but when only 1.2 moles of peroxybenzoic acid is used, there is obtained a mixture of the sulfoxide compounds (XVIII) and (XIX):

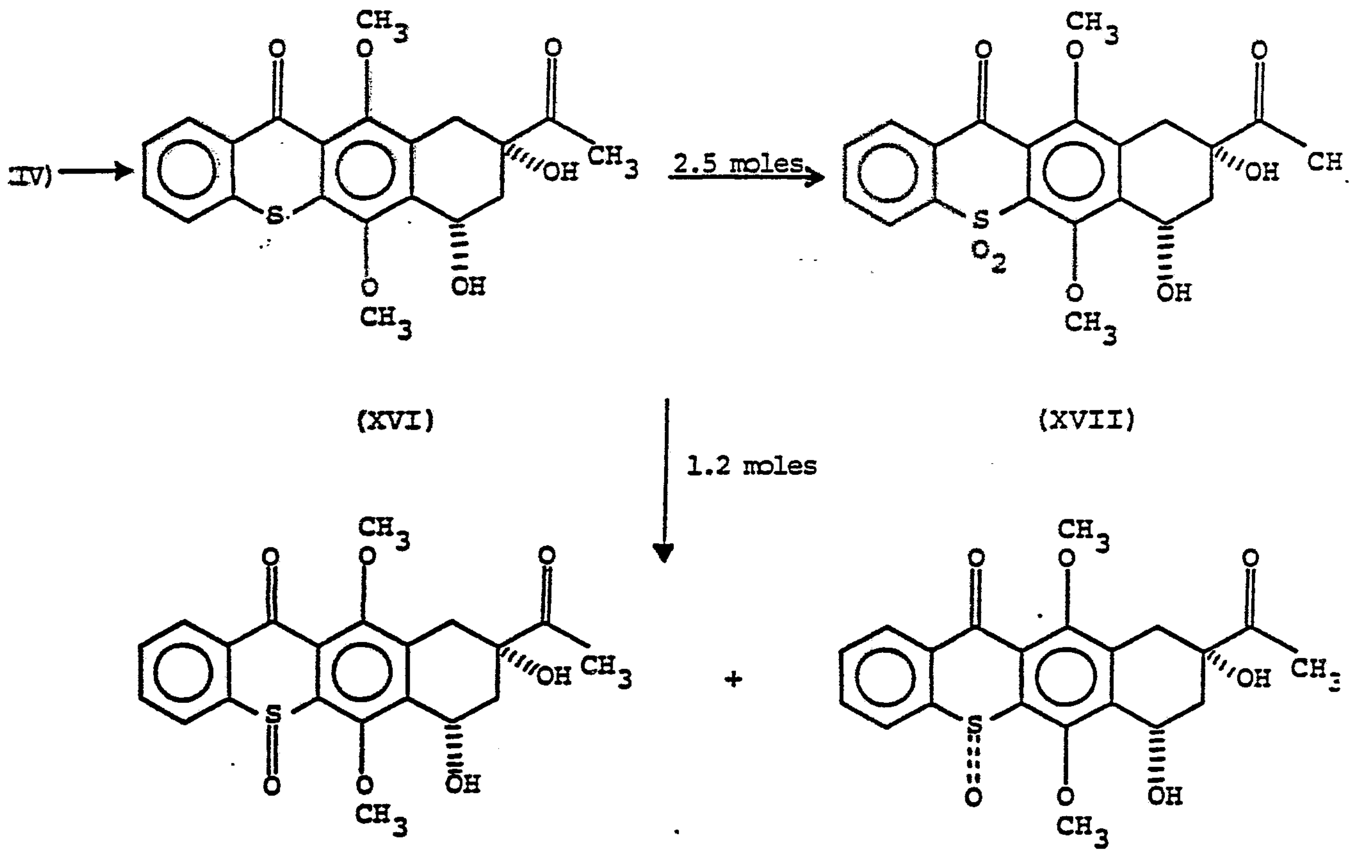


(XVI)

(XVII)

(XVIII)

(XIX)

The sulfono compound (XVII) and the sulfoxide compounds (XVIII) and (XIX) can be selectively demethylated by means of aluminum chloride in 1,2-dichloroethane. When using more than 10 folds of aluminum chloride, each compound is demethylated at both positions 6 and 11 whereas when using only 3 folds of aluminum chloride, each compound is demethylated only at position 11. Thus, the sulfono compound (XVII) can be demethylated to compounds (XX) and (XXI), the sulfoxo compound (XVIII) can be demethylated to compounds (XXII) and (XXIII) while the sulfoxo compound (XIX) can be demethylated to compounds (XVIV) and (XXV):

XVII)

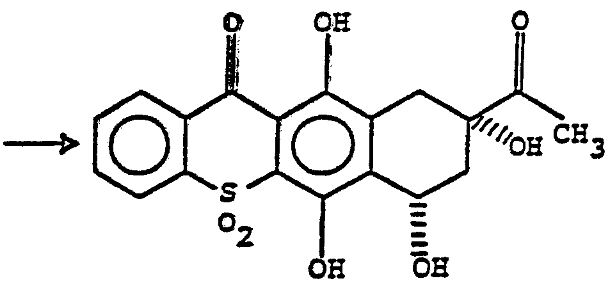

+

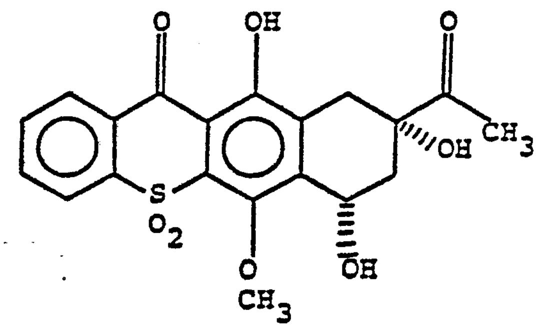

(XX) (XXI)

XVIII)

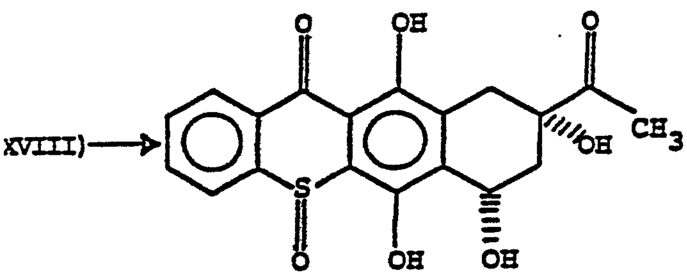

+

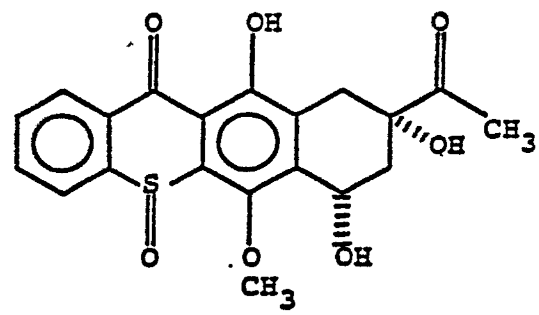

(XXII) (XXIII)

XIX)

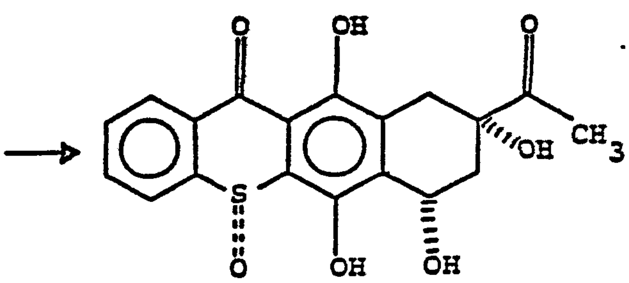

+

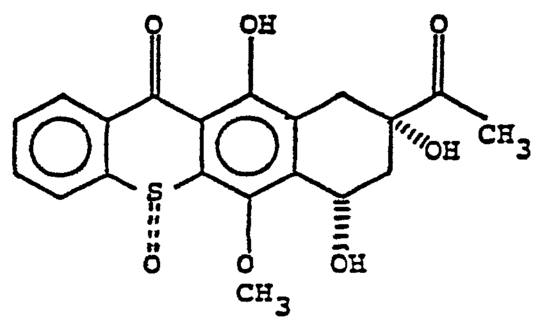

(XXIV) (XXV)

With respect to the geometric isomer, compound (XV), conversion to the corresponding sulfono compound or the two corresponding sulfoxo compounds can be achieved by the following procedure. Compound (XV) in a solution of tetrahydrofuran and ammonium hydroxide when treated with zinc dust and copper sulfate gives the corresponding compound (XXVI) which was dissolved in methanolic solution containing 2,2-dimethoxypropane to provide the compound (XXVII). The latter can then be oxidized with gaseous oxygen in the presence of potassium t-butoxide and trimethyl phosphite in a mixture of dimethylformamide and t-butanol to give the compound (XXVIII):

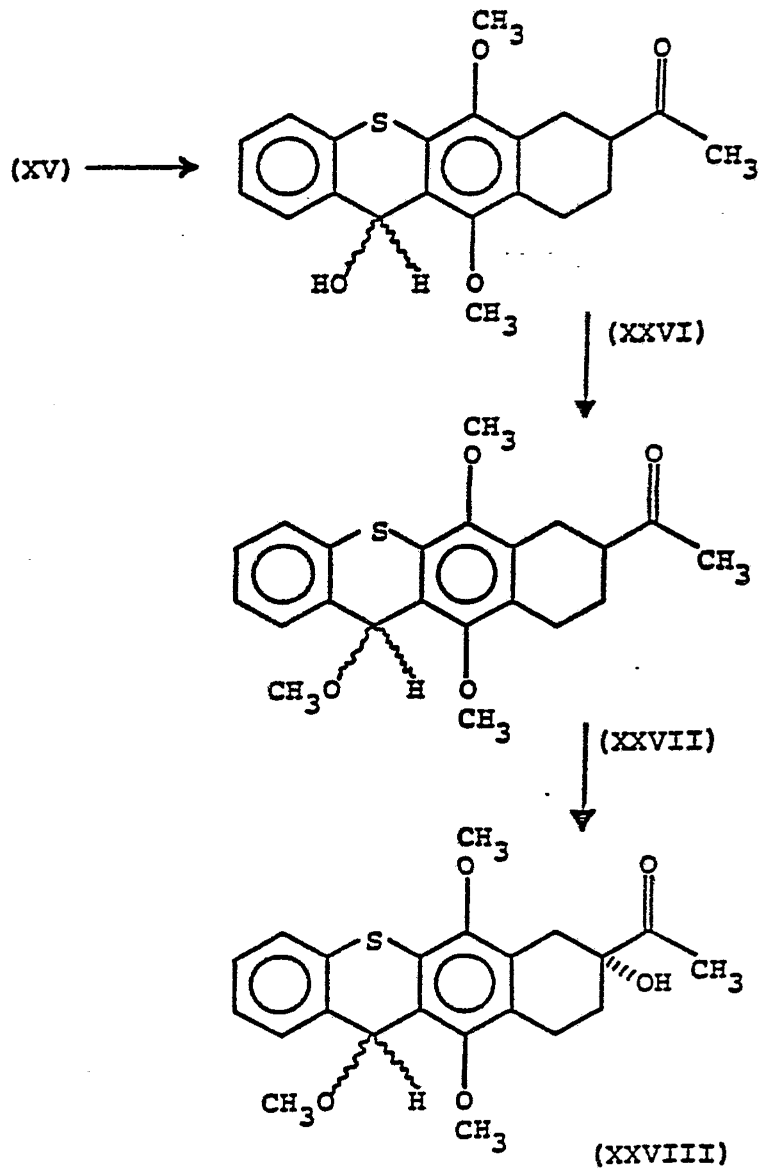


(XXVIII)

Compound (XXVIII) can be further oxidized, for example with peroxybenzoic acid or with a mixture of selenium dioxide and hydrogen peroxide. When using 2.2 moles of peroxybenzoic acid, there is obtained the sulfono compounds (XXIXa) and (XXIXb) but when only 1 mole of peroxybenzoic acid is used, there is obtained a mixture of the sulfoxo compounds (XXX) and (XXXI):

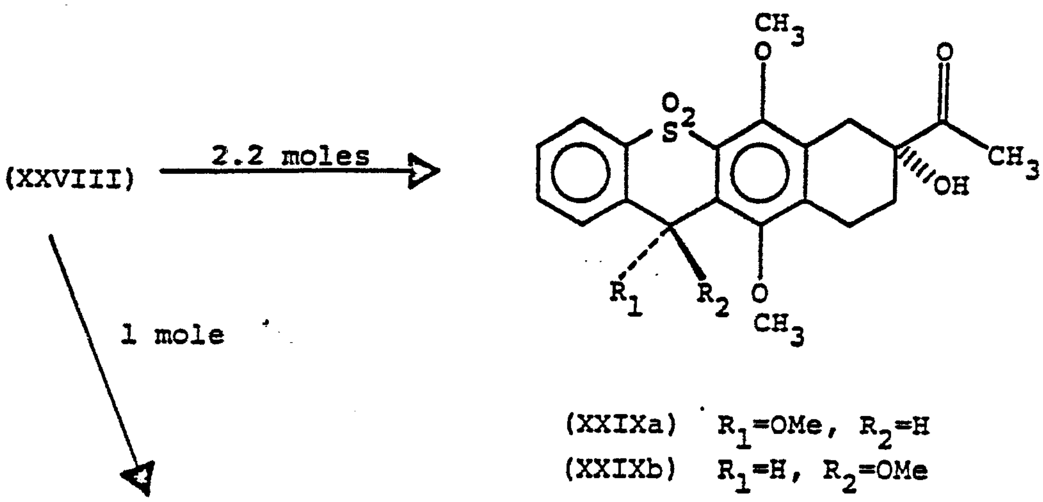


(XXIXa) $R_1$=OMe, $R_2$=H
(XXIXb) $R_1$=H, $R_2$=OMe

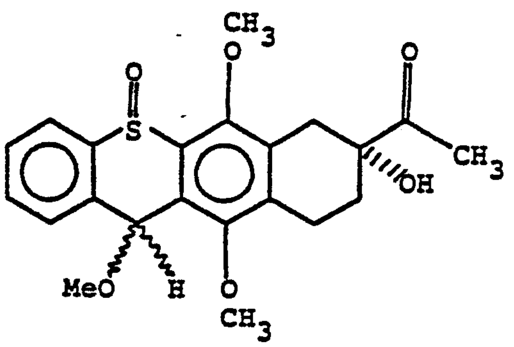

+

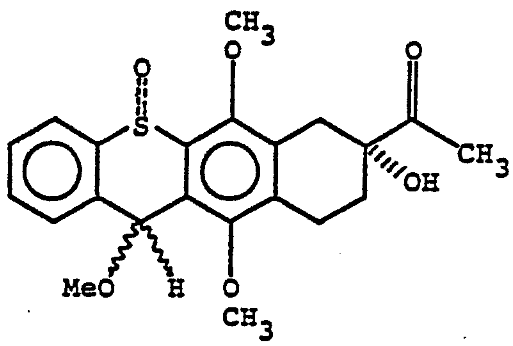

(XXX) (XXXI)

The sulfono compounds (XXIXa) and (XXIXb) can be converted to the desired aglycone by the following procedure: Treatment with trifluoroacetic acid gives the corresponding ester (XXXII) and subsequent reaction with methanol and potassium carbonate gives the alcohol (XXXIII) which when oxidized with manganese dioxide in dichloromethane provides the ketone (XXXIV):

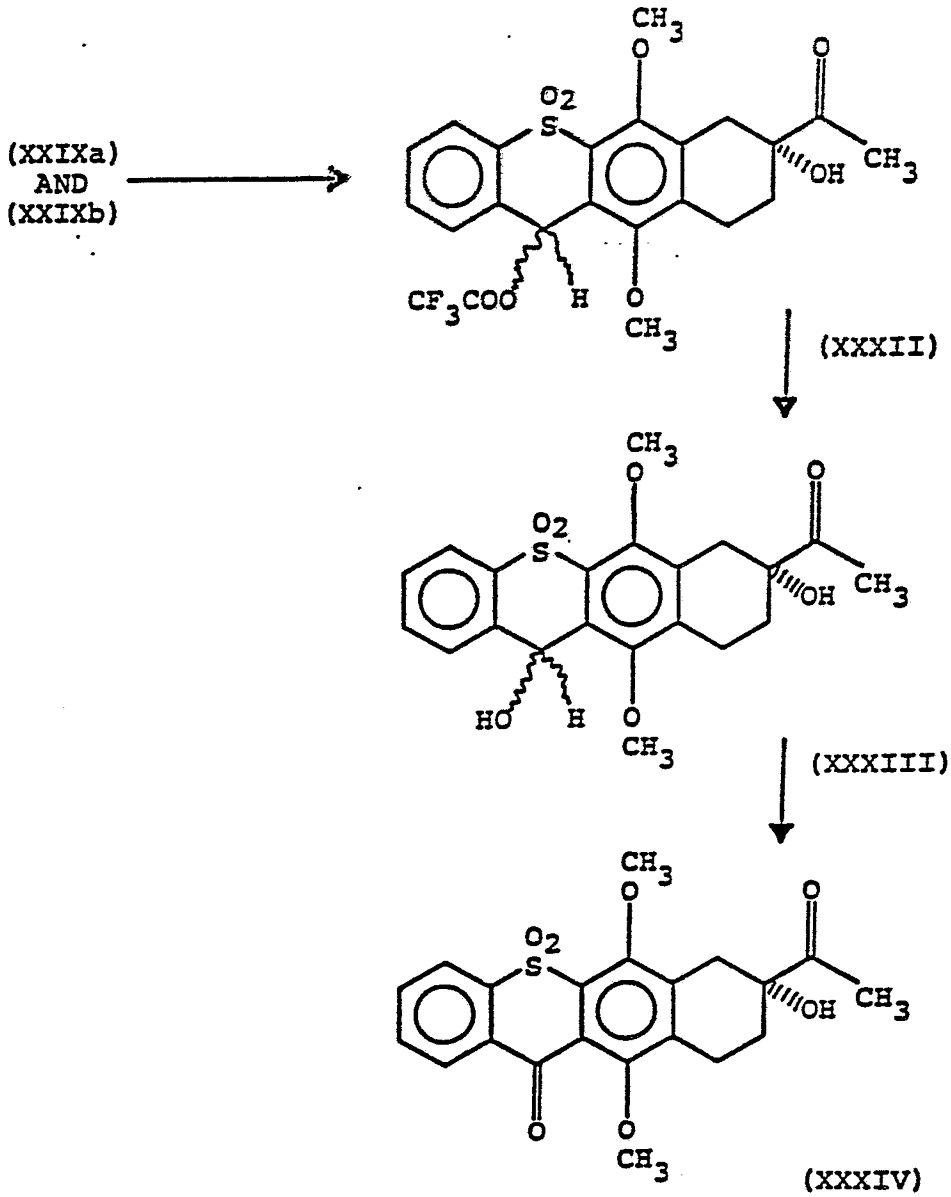

(XXIXa) AND (XXIXb)

(XXXII)

(XXXIII)

(XXXIV)

Introduction of the desired hydroxy group into position 7 of the tetracyclic structure can be carried out in the following stages. Irradiation of compound (XXXIV) in the presence of N-bromosuccinimide gave the bromo-compound (XXXV) which, when treated with methanol in the presence of silver trifluoromethane sulfonate, gave the 7-methoxy compound (XXXVI). Heating the latter with trifluoroacetic acid and subsequent hydrolysis of the product thus formed with potassium carbonate in aqueous methanol gave the 7-hydroxy compound (XXXVII):

(XXXIV)

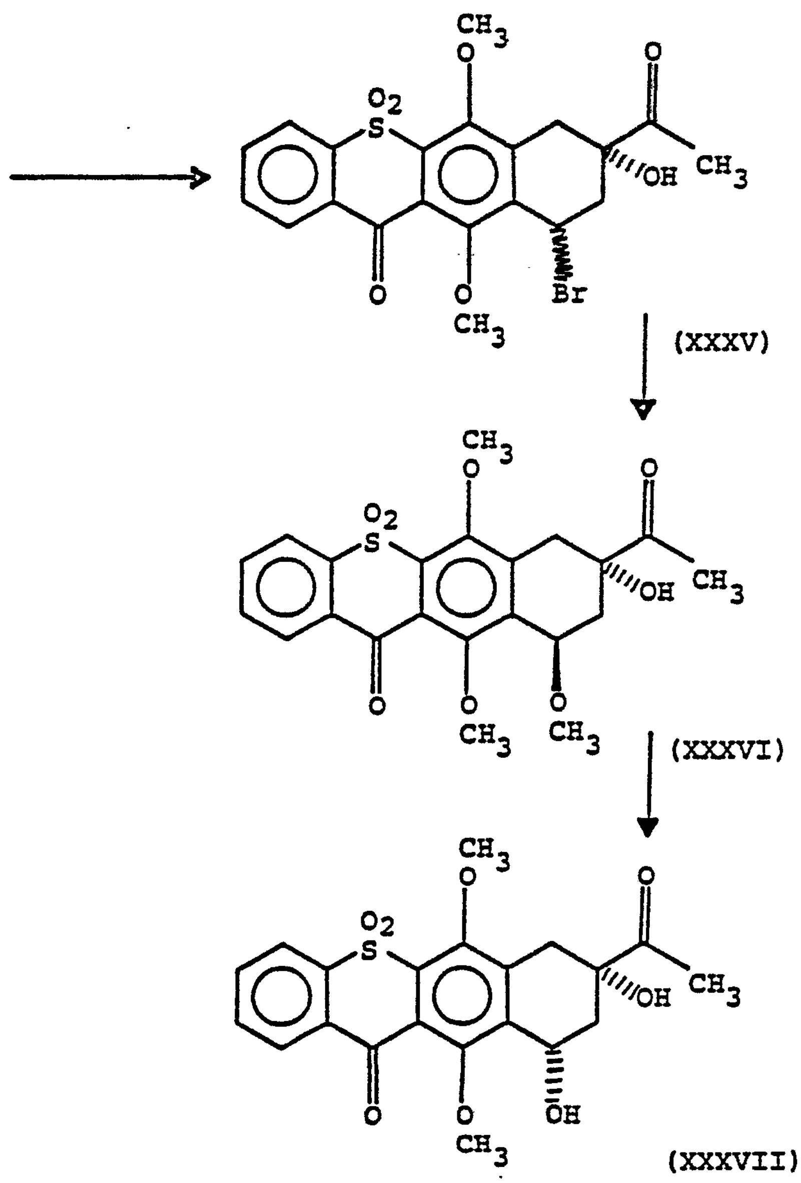


(XXXVII)

Selective demethylation of compound (XXXVII) by means of aluminum chloride gave the two desired aglycones. The use of more than 10 folds of aluminum chloride provided the 6,11-dihydroxy aglycone (XXXVIII) whereas the use of 3 folds of aluminum chloride provided the 6-hydroxy-11-methoxy aglycone (XXXIX):

(XXXVII)

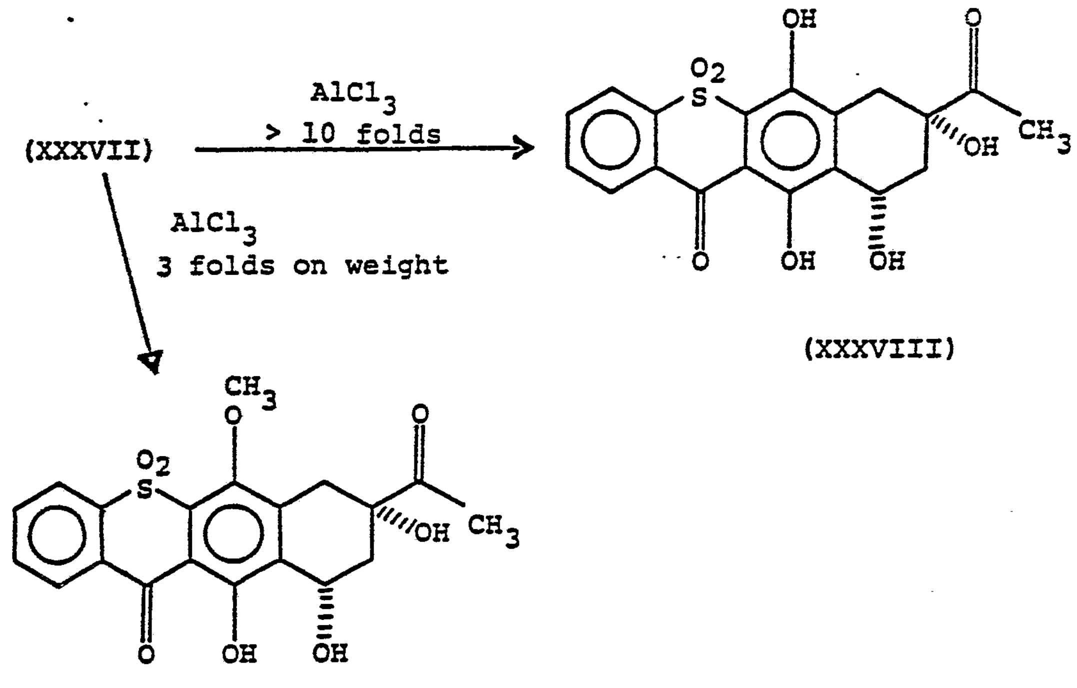


(XXXVIII)

(XXXIX)

In a similar manner, proceeding via steps of oxidation and demethylation, the intermediate compounds (XXX) and (XXXI) provide four more aglycones of the formulae (XL), (XLI), (XLII) and (XLIII) given below:

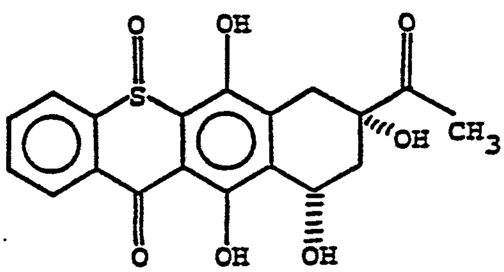

(XL)

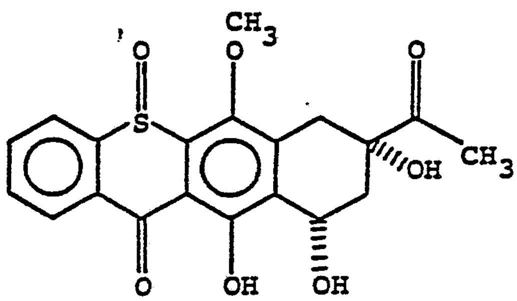

(XLI)

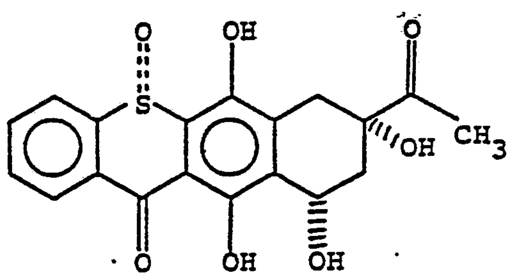

(XLII)

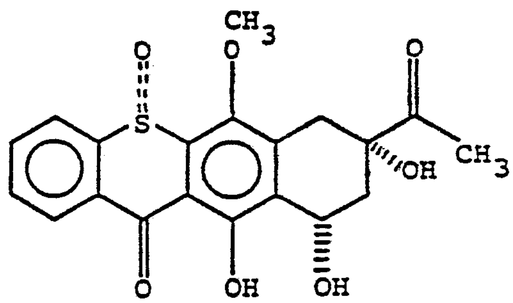

(XLIII)

Compounds I through X in free base form may be converted to pharmaceutically acceptable acid addition salts by treatment with a stoichiometric excess of the appropriate organic or inorganic acid, such as, for example, phosphoric, pyruvic, hydrochloric or sulfuric acid or the like. Typically, the free base is dissolved in a polar organic solvent such as ethanol or methanol, and the acid added thereto. The temperature is maintained between about 0°C and about 100°C. The resulting acid addition salt precipitates

spontaneously or may be brought out of solution with a less polar solvent. Such acid addition salts offer the advantage of being soluble in water and aqueous mixed solvents such as water alkanols or water alkandiols. Examples of these mixed solvents are water-propylene glycol, water-ethanol, water-ethylene glycol, saline, various other aqueous injectable media, and the like. The free bases are soluble in less polar organic solvents such as chloroform, methylene chloride, mixed chloroform-methanol solvents, and the like. They may also be used as suspensions.

The salts are the acid addition products of the free bases with a pharmaceutically acceptable acid. A "pharmaceutically acceptable" acid is one which is nontoxic and generally employed in pharmaceutical products. Examples of these acids are inorganic acids such as hydrochloric, hydrobromic, sulfuric and phosphoric acids, and organic acids such as the carboxylic acids, e.g., acetic, glycolic, maleic, malic, hydroxymaleic, tartaric, citric, and salicylic acids and the organosulfonic acids, e.g., methanesulfonic and p-toluenesulfonic acid. Mixtures of two or more acids may be used as may mixtures of one or more free bases plus one or more acid addition salts. For reasons of simplicity and ready solubility, the hydrochloric and hydrobromic acid additon salts are preferred.

The acid addition salts of the compounds of Formulae I-X may be converted to the corresponding free base by treatment with a stoichiometric excess of a suitable base, e.g., potassium carbonate or sodium hydroxide, typically in the presence of an aqueous solvent, and at a temperature of between about 0°C and about 100°C. The free base form is isolated by conventional means, such as by extraction with an organic solvent.

The compounds of the invention, including the salts thereof, can be administered by any available route, including oral and parenteral (intravenous, intraperitoneal, subcutaneous, and intramuscular) administration. Parenteral administration, especially intravenous administration, has historically been the mode of choice for analogous drugs and is preferred. For parenteral administration, the compound is dissolved or suspended in a suitable injectable liquid medium as is known in the art. Depending on the intended mode of administration, the compositions used may be in the form of solid, semi-solid or liquid dosage forms, such as, for example, tablets, suppositories, pills, capsules, powders, liquids, suspensions or the like, preferably in unit dosage forms suitable for single administration of precise dosages. While the compounds may be administered per se, it is more common to administer them in conjunction with a pharmaceutically acceptable carrier which dilutes the compound and facilitates handling. The term "pharmaceutically acceptable" in this context means that the carrier (as well as the resulting composition) is sterile and nontoxic. These compositions may also include other medicinal agents, pharmaceutical agents, carriers, and the like.

More specifically, for solid compositions, conventional nontoxic solid carriers include, for example, pharmaceutical grades of mannitol, lactose, starch, magnesium stearate, sodium saccarin, talcum, cellulose, glucose, sucrose, magnesium carbonate, and the like. The active compound as defined above may be formulated as suppositories using, for example, polyalkylene glycols as the carrier. Liquid pharmaceutically acceptable compositions can, for example, be prepared by dissolving, dispersing, etcl, an active compound as defined above and optional pharmaceutical adjuvants in a carrier, such as, for example, water, saline, aqueous dextrose, glycerol, ethanol, and the like, to thereby form a solution or suspension. If desired, the pharmaceutical composition to be administered can also contain minor amounts of nontoxic auxiliary substances such as wetting or emulsifying agents, pH buffering agents and the like, for example, sodium acetate, sorbitan monolaurate, triethanolamine sodium acetate, triethanolamine oleate, etc. Actual methods of preparing such dosage forms are known, or will be apparent to those skilled in the art; reference may be had to Remington's Pharmaceutical Sciences, Mack Publishing Company, Easton, Pennsylvania, 15th Edition, 1975. In the preparation of these dosage forms, one can use the art-accepted techniques for formulating water-soluble pharmaceutical agents (in the case of salts) and water-insoluble agents (in the case of the free bases).

For oral administration, a pharmaceutically acceptable nontoxic composition is formed by the incorporation of any of the normally employed excipients, such as, for example, pharmaceutical grades of mannitol, lactose, starch, magnesium stearate, sodium saccharin, talcum, cellulose, glucose, sucrose, magnesium, carbonate and the like. Such compositions can take the form of solutions, suspensions, tablets, pills, capsules, powders, sustained release formulations and the like. Such compositions typically contain 10% to 95% active ingredient.

A more recently devised approach for parenteral administration employs the implantation of a slow-release or sustained-release system, such that a constant level of dosage is maintained.

For systemic administration via suppository, traditional binders and carriers include, e.g., polyalkylene glyhcols and triglycerides. Such suppositories may be formed from mixtures containing active ingredient in the range of from about 0.5% to about 10%, more preferably in the range of from about 1% to about 2%.

The amount of active compound administered will, of course, be dependent on the subject being treated, the manner of administration, and the judgment of the prescribing physician. However, the dosing regimen and amount administered is sufficient to ameliorate the leukemia or other type of cancer against which the compounds hereof are effective. For example, in the treatment of lower test animals, a dosage of a compound of the present invention within the range from about 0.0010 mg/kg to about 25 mg/kg per day should be sufficient to ameliorate leukemia. The upper dosage limit is that imposed by toxic side effects and can be determined by trial and error for the animal to be treated. Dosing regimens of one dose every 2 to 7 days are effective while shorter intervals, say one day or less, between dosings may be used as well.

The invention is illustrated, but not limited by the following examples describing the preparation of compounds (III), (IV), (V), (VI) and (XLVII).

EXAMPLE 1

To an ice cooled solution of 1,2-dichloroethane (250 ml) containing 2-acetyl-5,8-dimethoxytetralin (13.4 g) and 2-chlorosulfenylbenzoyl chloride (20 g) was added stannic chloride (4 ml). The solution was stirred in an oil-bath at 50°C under nitrogen and anhydrous conditions for 2 hours, and then cooled in an ice-bath and washed with water. The organic layer was further washed with saturated oxalic acid solution, sodium bicarbonate solution and finally water. After drying over magnesium sulfate, the organic layer was evaporated to dryness under reduced pressure and the residue (25 g) was purified, and compounds (XIV) (10.7 g) (m.p. 160 to 162°C) and (XV) (6.4 g) (m.p. 142 to 144°C) were separated by column chromatography over silica in a ratio of 3:2. When the reaction was carried out in concentrated sulfuric acid using 2,2'-dithiobenzoic acid instead of 2-chlorosulfenylbenzoyl chloride, the product ratio was reversed. The tetracyclic compound (XV) (6 g) was dissolved in a solution of tetrahydrofuran (THF) (200 ml) and ammonium hydroxide (200 ml). To the solution was added zinc dust (40 g) and copper sulfate (1 g) and the mixture was stirred at room temperature for 45 minutes. The zinc dust was removed by filtration, and washed thoroughly with acetone. The acetone solution was concentrated and combined with the THF solution. The combined solution was diluted with water and extracted with dichloromethane exhaustively.

The combined extracts, dried over magnesium sulfate, were evaporated to dryness under reduced pressure to give (XXVI) in quantitative recovery. The product was then dissolved in anhydrous methanol containing 2,2-dimethoxypropane and heated under reflux for 8 hours. Evaporation of the solution to dryness under reduced pressure gave (XXVII) in quantitative recovery as a light yellow foam.

The foamy product (XXVII) was dissolved in a solution of anhydrous dimethylformamide (250 ml) and t-butyl alcohol (35 ml). To the solution, cooled in an ice-bath at -30°C, was added potassium t-butoxide (6 g) and trimethyl phosphite (7 ml). Dry oxygen was introduced into the solution through a gas dispersion tube for 1/2 hour and then neutralized to pH = 7 to 8 by dilute HCl. Stirring was continued for an hour at room temperature. The solution was further diluted with water and extracted exhaustively with dichloromethane. Evaporation of the dichloromethane after the usual procedure gave a brown residue which was purified by column chromatography on silica to give (XXVIII) as an amorphous solid which could be separated into $C_5$-$\alpha$-OMe and $C_5$-$\beta$-OMe isomers. Separation was not necessary except for characterization purpose, because oxidation of both isomers gave the same compound (XXXIV). (vide infra).

Thus to a chloroform solution (250 ml) of (XXVIII) (6 g) cooled in an ice-bath was added m-chloroperoxybenzoic acid (5.6 g) and the solution was stirred at room temperature for one hour, washed with 2% $Na_2CO_3$ solution, dried and evaporated to dryness as usual. The major product (XXIXa) and (XXIXb) (5.3 g) was purified by chromatography over silica. Careful separation by thin layer chromatography over silica for identification purpose gave (XXIXa) m.p. 234 to 237°C and (XXIXb) m.p. 228 to 231°C. A trifluoroacetic acid solution of (XXIXa) and (XXIXb) (4 g) was stirred at room temperature until the initially dark solution became clear. The trifluoroacetic acid was removed by evaporation under reduced pressure, and the residue (XXXII) was stirred at room temperature in methanol (100 ml) and saturated potassium carbonate solution for 15 hours. The solution was diluted with water and extracted exhaustively with chloroform. The chloroform extract was evaporated to dryness as usual. The residue (XXXIII) was dissolved in dichloromethane and was oxidized by manganese dioxide (40 g) to give the crude (XXXIV) (2.3 g) which was purified by column chromatography on silica; m.p. 194 to 196°C.

To a carbon tetrachloride solution of (XXXIV) (2 g) (200 ml) heated under reflux and irradiated by a 500 watt quartz iodine light, was added N-bromosuccinimide (0.9 g) and the reaction was allowed to proceed for 45 minutes. The solid succinimide was removed by filtration, washed with hot carbon tetrachloride and the combined carbon tetrachloride solution was evaporated as usual. The residue containing the unstable product (XXXV) was purified by fast column chromatography on silica and then converted to (XXXVII) (1.4

g) by treating with methanol in the presence of silver trifluoromethanesulfonate. Heating (XXXVI) in trifluoroacetic acid in a 60°C oil-bath for 6 hours followed by evaporation to dryness and hydrolysis of the residue in potassium carbonate aqueous methanolic solution gave (XXXVII) (1.27 g) (m.p. 171 to 174°C). Demethylation of (XXXVII) by aluminum chloride in chloroform solution gave the aglycone (XXXVIIII) or (XXXIX) depending on the quantity of aluminum chloride. Thus the sulfone (XXXVII) (800 mg) in 1,2-dichloroethane solution (100 ml) was allowed to react with freshly sublimed $AlCl_3$ (10 g) in the presence of glass bead. The solution was stirred for 20 hours at room temperature, diluted with more chloroform (100 ml) and extracted with saturated oxalic acid solution (4 x 100 ml), washed with water, dried over anhydrous sodium sulfate and evaporated to dryness yielding an orange solid which was purified by column chromatography over acid washed silica to give the (XXXVIII) (250 mg) (m.p. 222 to 225°C). When the sulfone (XXXVII) (400 mg) was allowed to react with $AlCl_3$ (1.5 g) under similar condition, (XXXIX) was isolated as the only major product (280 mg) (m.p. 206 to 209°C).

The Koenig-Knorr condensation for the synthesis of (IV) was carried out according to the following - scheme::

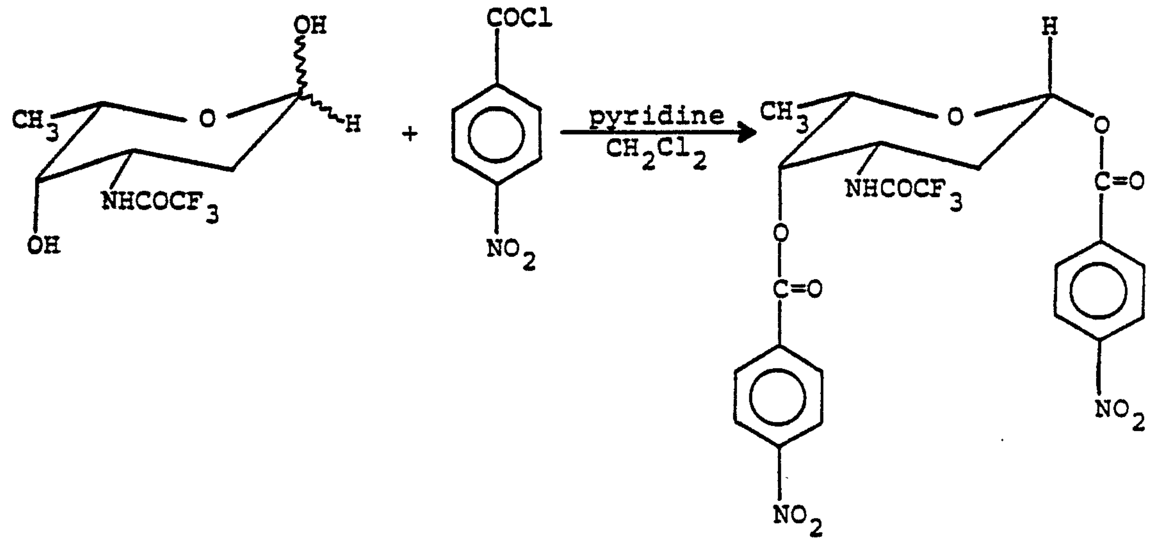


(XLIV)

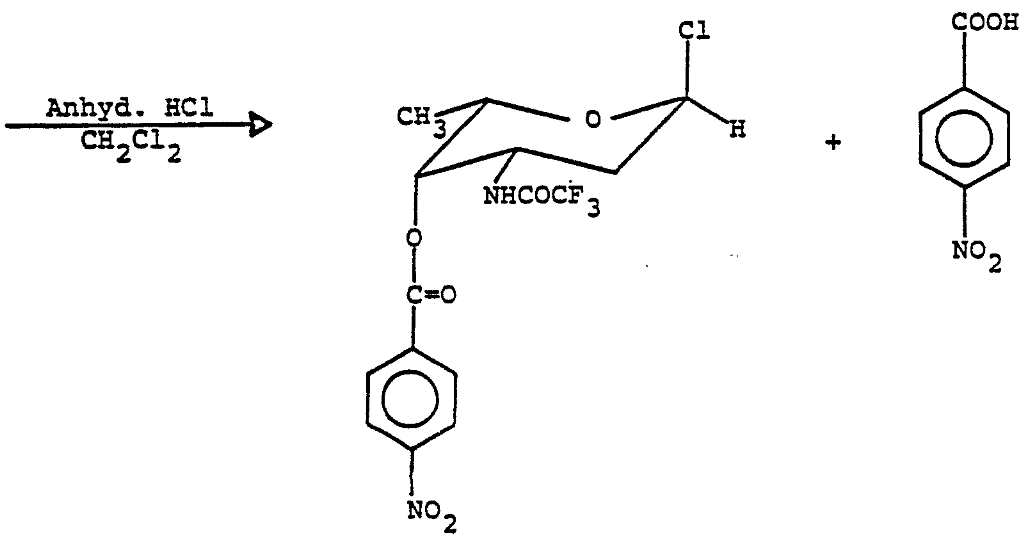


(XLV)

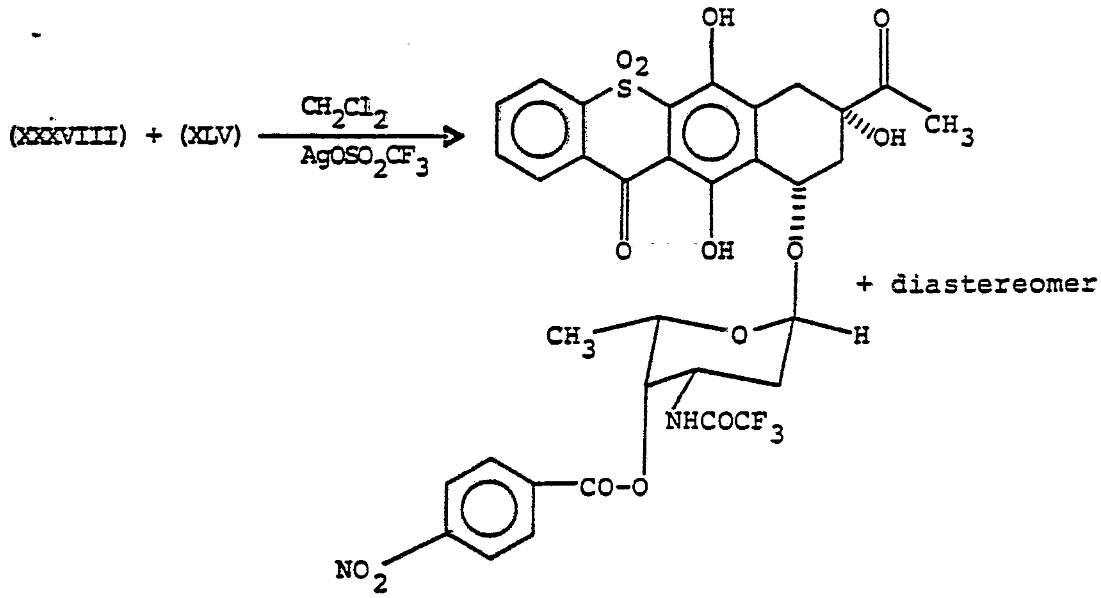


(XLVI)

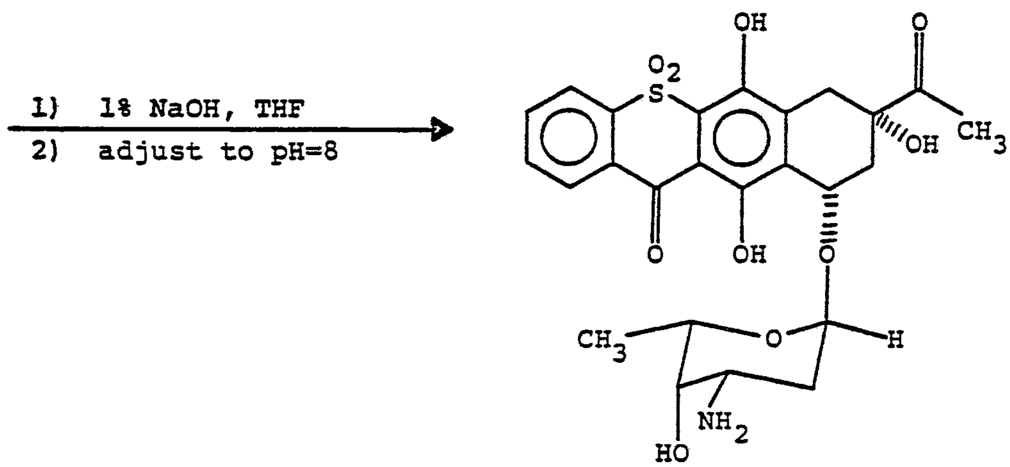


(IV)

N-trifluoroacetyl daunosamine (2.4 g) was allowed to react with p-nitrobenzoyl chloride (4.8 g) in methylene chloride solution (100 ml) in the presence of pyridine (7 ml). The solution was stirred at room temperature for 15 hours, evaporated to dryness under reduced pressure and the residue was redissolved in dichloromethane (25 ml) to which anhydrous ether (100 ml) was then added. The precipitate was removed by filtration, dissolved again in dichloromethane and precipitated once more by the addition of anhydrous ether. The process was repeated once more so that the precipitate when analyzed by thin layer chromatography should be free of the product (XLIV). The combined filtrates were evaporated to dryness as usual and (XLIV) (4.9 g) was isolated very easily by fast column chromatography on silica from the by-product p-nitrobenzoic anhydride and the starting material p-nitrobenzoyl chloride.

The crystalline (XLIV) (m.p. 193 to 195°C) (810 mg) was dissolved in anhydrous dichloromethane (50 ml) and cooled in an ice-bath. Anhydrous hydrogen chloride was introduced into the solution through a gas dispersion tube and the solid p-nitrobenzoic acid precipitated out, was removed by filtration. The filtrate was evaporated to dryness, redissolved in fresh dichloromethane and again evaporated to dryness until the foamy residue was free of residual hydrogen chloride. The whole operation was carried out under absolutely

anhydrous conditions.

To this residue was added (XXXVIII) (400 mg), anhydrous dichloromethane (70 ml), pulverized freshly baked molecular sieve (2 g) and a tetrahydrofuran solution (10 ml) of silver trifluoromethanesulfonate (150 mg). The reaction mixture was filtered after 5 hours of stirring at room temperature in darkness. The residue was washed with dichloromethane and the com bined filtrates were washed with NaCl solution, dried over anhydrous sodium sulfate and evaporated to dryness as usual. Column chromatography on silica gave (XLVI) separated from its diastereomer. The protecting trifluoroacetyl and p-nitrobenzoyl groups were removed from compound (XLVI) by treatment with aqueous sodium hydroxide solution (20 ml 1%) in tetrahydrofuran (20 ml). The reaction mixture was adjusted to pH 8.0 by dilute hydrochloric acid and extracted exhaustively by chloroform. The chloroform extract, dried over anhydrous sodium sulfate was evaporated to dryness under reduced pressure to give a dark orange coloured residue from which pure (IV) m.p. 162 to 170°C (dec) was isolated by column chromatography on silica. All new compounds are characterized by infrared, high resolution FT pmr or 2-D pmr spectrum or by molecular ion measurement or by high resolution mass spectrometer or by elemental analysis.

## EXAMPLE 2

The intermediate (XVI) (820 mg) was allowed to react with m-chloroperoxybenzoic acid (1:1.2 mol. ratio) in methylene chloride solution for 2/3 hour at room temperature. The methylene chloride solution was washed with (5%) sodium carbonate solution, dried over anhydrous sodium sulfate and evaporated to dryness. Chromatography of the dark orange residue on silica gave 2 isomeric sulfoxides (XVIII) (m.p. 195 to 198°C) and (XIX) (m.p. 213 to 215°C). Demethylation was performed in the following manner:

To the chloroform solution (50 ml) of (XVIII) (200 mg) was added aluminum chloride (3 g ) and the solution was stirred in a 50°C oil-bath for 17 hours. The solution was diluted with more chloroform and then washed several times with saturated oxalic solution, dried over anhydrous sodium sulfate and evaporated to dryness to yield a red semi-solid residue from which was isolated (XXII) (77 mg) (m.p. 197 to 201°C) and (XXIII) (35 mg) (m.p. 194 to 197°C). Demethylation of (XIX) under similar conditions gave (XXIV) (m.p. 201 to 203°C) and (XXV) (m.p. 196 to 199°C).

Reaction of (XX) with (XLV) under conditions identical to the preparation of (IV) led to the synthesis of (III). Reaction of (XXII) with (XLV) under conditions identical to the preparation of (IV) led to the synthesis of (V) and reaction of (XXV) with (XLV) likewise gave (VI). All compounds were characterized by infrared, proton magnetic resonance and mass spectra.

## EXAMPLE 3

Selective demethylation of (XVII) to (XXI), (XVIII) to (XXIII) and (XIX) to (XXV) were performed under similar condition as illustrated in the following process:

Compound (XVIII) (416 mg) in chloroform (40 ml) was allowed to react with aluminum chloride (1.5 g) at room temperature for 15 hours. Additional chloroform (150 ml) was added and the solution was washed with saturated oxalic acid solution (3 x 50 ml), dried over sodium sulfate and evaporated to dryness as usual. The main product (XXIII) was isolated by column chromatography over silica and recrystallized from methylene chloride acetone (300 mg) (m.p. 194 to 197°C).

Reaction of (XXIII) with (XLV) under condition similar to the preparation of (IV) gave the intermediate (XLVII) (m.p. 182 to 186°C) which underwent basic hydrolysis, as detailed in Example 1, and gave (IX) as an amorphous solid (m.p. 160 to 168°C) (dec) which was converted to the corresponding hydrochloride by anhydrous hydrogen chloride and precipitated from cold methylene chloride solution (m.p. 181 to 186°C).

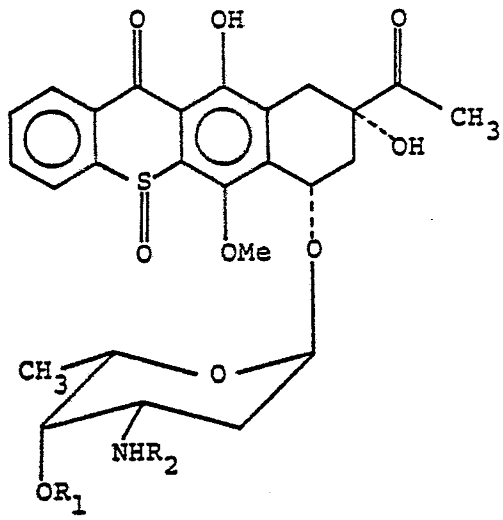

(XLVII) $R_1$=p-nitrobenzoyl
$R_2$=trifluoroacetyl

(IX) $R_1=R_2=H$

EXAMPLE 4

The compounds of this invention have utility as mammalian antitumor agents. This activity is evidenced by in vivo and in vitro studies. In one in vivo test, conducted in accordance with the protocol described in Cancer Chemotheraphy Reports, National Cancer Institute 3 (2), Part 3, September 1972, healthy mice are inoculated i.p. with Lymphocyte Leukemia P-388 ascitic fluid. The inoculated mice are then treated on days 5, 9 and 13 of the succeeding period with various amounts of compounds of the invention. For purposes of comparison, other mice are untreated and additional mice are treated with daunorubicin or doxorubicin.

The average survival time of the various treated mice is determined and compared with that of the mice inoculated withe the leukemia ascitic fluid but given no treatment with the test compound. The results show that compounds of this invention have superior in vivo antitumor activity at low optimum dosages. This gives promise of providing an active antitumor agent with substantially decreased cardiotoxicity.

In vitro tests of the compounds of the invention also show increased activity. When these materials are tested as inhibitors of DNA and RNA synthesis in P-388 leukemia cells by the method described in G. Tong, W.W. Lee, D.R. Black and D.W. Henry, J. Medicinal Chem. 19: 395 (1976), they are active at doses that were far lower than the doses of daunorubicin or doxorubicin known in the art.

## Claims

1. A process for the preparation of a novel compound of the formula (I):

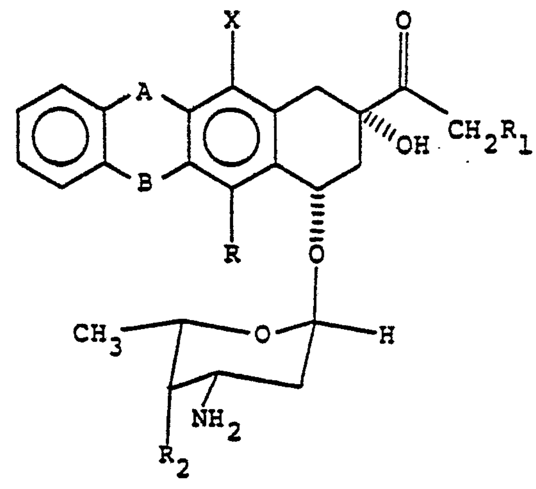

(I)

including all stereoisomers thereof, or a pharmaceutically acceptable salt thereof, wherein, of A and B, one is C=O and the other us S≈Y; R is hydrogen, hydroxy or methoxy; $R_1$ is hydrogen or hydroxy; $R_2$ is hydrogen or hydroxy; X is hydrogen, hydroxy or methoxy and Y is O or $O_2$; provided that R and X do not both stand for methoxy, which comprises reacting an aglycone of the formula:

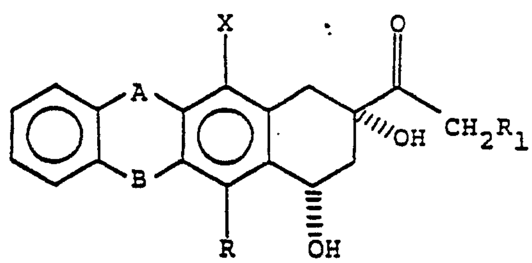

with a daunosamine derivative of the formula:

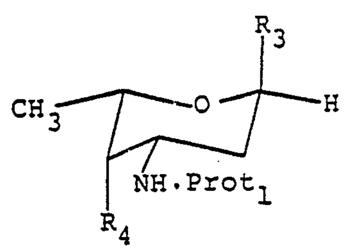

wherein A, B, R, $R_1$, X and Y have the meaning stated above, $R_3$ is a leaving group and $R_4$ is hydrogen or O•$Prot_2$, wherein $Prot_1$ and $Prot_2$ are protecting groups and substantially removing any protecting group or groups to provide the final product.

2. The process of claim 1 wherein the reaction is carried out in the presence of an organic diluent or solvent in the presence of silver trifluoromethanesulfonate.

3. The process of claim 1 or 2 wherein the removal of any protecting groups or groups is effected by treatment with an alkaline medium.

4. The process of claim 3 wherein the treatment is carried out in the presence of an organic diluent or solvent.

5. The process of any one of the preceding claims wherein the leaving group $R_3$ is chlorine.

6. A novel aglycone of the formula:

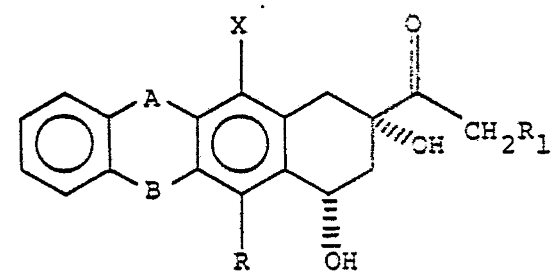

wherein, of A and B, one is C=O and the other is S≋Y; R is hydrogen, hydroxy or methoxy; $R_1$ is hydrogen or hydroxy; $R_2$ is hydrogen or hydroxy; X is hydrogen, hydroxy or methoxy and Y is O or $O_2$.

7. A novel compound of the formula (I):

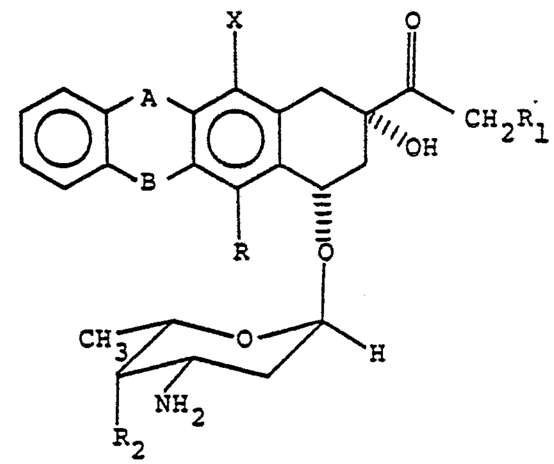

(I)

including all stereoisomers thereof, or a pharmaceutically acceptable salt thereof, wherein, of A and B, one is C=O and the other is S≋Y; R is hydrogen, hydroxy or methoxy; $R_1$ is hydrogen or hydroxy; $R_2$ is hydrogen or hydroxy; X is hydrogen, hydroxy or methoxy and Y is O or $O_2$; provided that R and X do not both stand for methoxy.

8. A compound which is selected from compounds of the formulae:

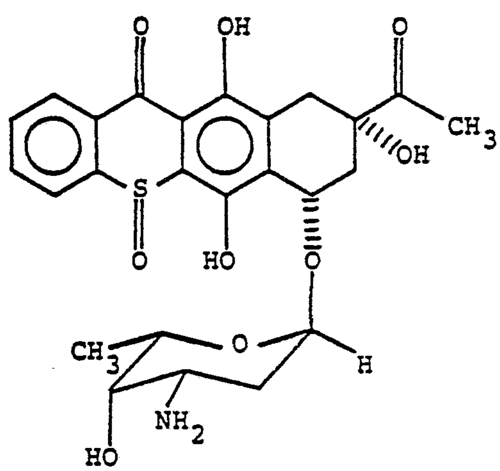

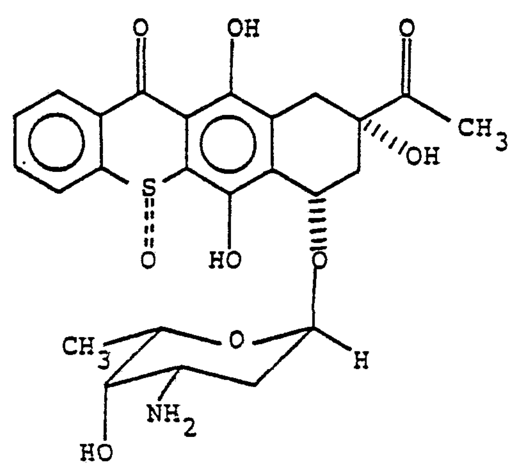

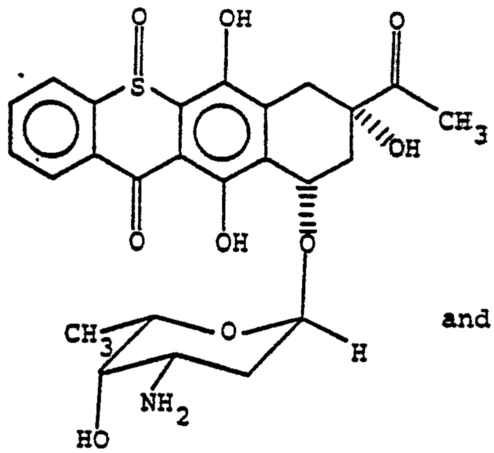

and

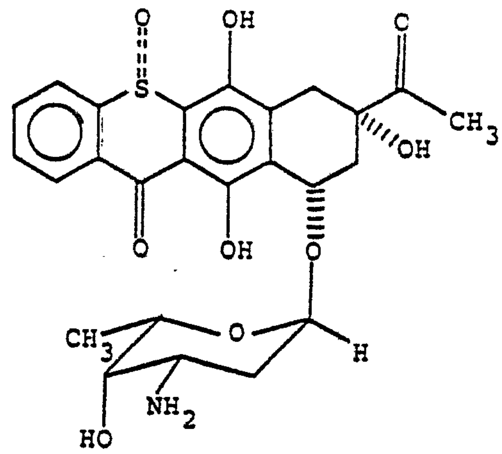

9. The compound of the formula:

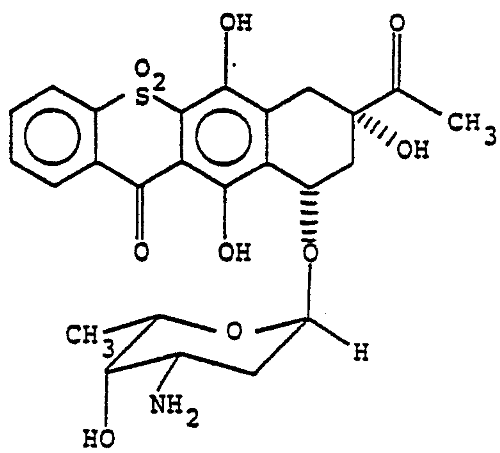

10. The compound of the formula: